# FASCICULE DE BREVET EUROPEEN

(11) **EP 0 632 002 B1**
(45) Date de publication et mention de la délivrance du brevet: **04.06.1997**
(21) Numéro de dépôt: 94401394.5
(22) Date de dépôt: 22.06.1994
(51) Int. Cl.: C07C 19/08, C09K 5/04

(54) **Mélanges non azéotropiques contenant du difluorométhane et du 1,1,1,2-tétrafluoroethane et leurs applications comme fluides frigorigènes en conditionnement d'air**
Nicht-azeotropische Difluoromethan und 1,1,1,2-Tetrafluoroethan enthaltende Zusammensetzungen und ihre Verwendung als Kühlflüssigkeiten für Klimaanlagen
Non azeotropic mixtures containing difluoromethane and 1,1,1,2-tetrafluoroethane, and their use as refrigerating fluids in air conditioning

(30) Priorité: 30.06.1993 FR 9307975
(43) Date de publication de la demande: 04.01.1995
(73) Titulaire: ELF ATOCHEM S.A., 92800 Puteaux (FR)
(72) Inventeur: Macaudiere, Sylvie, F-92600 Asnieres (FR)
(74) Mandataire: Leboulenger, Jean

(56) Documents cités:
- EP-A- 0 430 169
- EP-A- 0 430 171
- EP-A- 0 509 673
- WO-A-92/16596
- WO-A-92/16597

## Description

La présente invention concerne le domaine de la réfrigération et a plus particulièrement pour objet des mélanges non azéotropiques de fluides frigorigènes n'ayant pas ou peu d'action sur l'environnement, pour remplacer les chlorofluorocarbones (CFC) ou hydrochlorofluorocarbones (HCFC) dans les systèmes de conditionnement d'air.

Dans ces systèmes exploités en conditionnement d'air, on opère selon un cycle thermodynamique défini généralement par une température d'évaporation comprise entre 0 et 10°C (le plus souvent 7°C), une température de condensation comprise entre + 30 et 55°C, un sous-refroidissement liquide de l'ordre de - 5°C et une surchauffe des vapeurs d'au moins 10°C.

Le fluide frigorigène couramment utilisé en conditionnement d'air est le chlorodifluorométhane (appelé HCFC 22). Or, il est maintenant établi qu'à cause de leur coefficient d'action sur l'ozone, les HCFC et en particulier le HCFC 22 devront à plus ou moins longue échéance être remplacés par des fluides frigorigènes ne contenant plus de chlore et, de ce fait, moins agressifs vis-à-vis de l'environnement.

Pour remplacer le HCFC 22 dans les installations de conditionnement d'air existantes, le substitut doit présenter des propriétés thermodynamiques, en particulier un coefficient de performance (COP) et une capacité frigorifique, aussi proches que possible de celles du HCFC 22.

D'autre part, pour la bonne stabilité du produit et la durabilité du matériel, il est souhaitable que la température de refoulement n'excède pas celle du HCFC 22 de plus de 5°C environ. Enfin, le substitut doit être ininflammable et le rester en cas de fuite en phase vapeur.

Le difluorométhane (HFC 32), le tétrafluorométhane (FC 14), l'octafluoropropane (FC 218) et le 1,1,1,2-tétrafluoroéthane (HFC 134a) n'ont comparativement aux composés chlorés aucune action sur l'ozone et une très faible action sur l'environnement.

Le HFC 32 présente l'inconvénient majeur d'être inflammable. De plus son point d'ébullition largement plus bas que celui du HCFC 22 fait qu'il n'est pas adapté au remplacement direct du HCFC 22. Il est connu par l'homme de l'art qu'un bas point d'ébullition entraîne des pressions très élevées qui rendent impossible l'emploi d'un tel fluide dans les systèmes existants pour des raisons de sécurité.

Le HFC 134a, composé ininflammable, est un bon fluide frigorigène en terme d'efficacité, mais sa trop faible capacité frigorifique rend son emploi impossible comme substitut au HCFC 22.

Les mélanges des deux HFC cités précédemment dans certaines proportions conduisent à un bon compromis entre l'efficacité et la capacité frigorifique mais, dans ces proportions, les mélanges présentent une phase vapeur inflammable.

Il a été maintenant trouvé que les mélanges contenant en masse environ 26 à 34 % de HFC 32, environ 56 à 73 % de HFC 134a et environ 1 à 10 % d'hydrocarbure perfluoré FC 14 ou FC 218 présentent, comparativement aux composés individuels, des propriétés thermodynamiques très proches de celles du HCFC 22. Les mélanges selon l'invention possèdent de plus un coefficient d'action sur l'ozone nul et un effet de serre plus faible que celui du HCFC 22. Enfin, au contraire du HFC 32 et de certains mélanges de HFC 32/134a, les mélanges selon l'invention sont ininflammables à 25°C et le restent en cas de fuites de la phase vapeur.

Les mélanges selon l'invention peuvent donc être utilisés dans les systèmes de conditionnement d'air, notamment pour le remplacement du HCFC 22.

Parmi les mélanges selon l'invention, un mélange tout particulièrement préféré contient environ 32 % de HFC 32, 65 % de HFC 134a et 3 % de FC 218.

Les exemples suivants illustrent l'invention sans la limiter.

### EXEMPLE 1

Cet exemple montre l'évolution de la pression et de la composition d'un mélange HFC 32/HFC 134a/FC 218, au cours d'une fuite de la phase vapeur.

La température a été maintenue constante à 24°C. Les pressions ont été mesurées au moyen d'un manomètre Heise avec une précision supérieure à ± 1 %. Le récipient est initialement chargé avec environ 342 g d'un mélange contenant en masse 33 % de HFC 32, 64 % % de HFC 134a et 3 % de FC 218.

La fuite en phase vapeur est poursuivie jusqu'à ce que 70 % de la charge initiale soit dissipée. Durant l'expérience, des échantillons de la phase gaz sont collectés et analysés par les moyens standard de chromatographie phase gaz. La tension de vapeur est également mesurée au même moment. Les résultats obtenus sont rassemblés dans le tableau suivant.

**TABLEAU 1**

| **Taux de fuite %** | **Pression absolue bar** | **Composition % masse** | | |
|---|---|---|---|---|
| | | **HFC 32** | **HFC 134a** | **FC 218** |
| 0 | 12,04 | 46,2 | 43,4 | 10,4 |
| 7,4 | 11,76 | 47,6 | 42,6 | 9,8 |
| 18,8 | 11,42 | 45,3 | 47 | 7,7 |
| 37,9 | 10,93 | 45,2 | 48,7 | 6,1 |
| 49,3 | 10,39 | 39,3 | 57,1 | 3,6 |
| 60,8 | 9,83 | 34,2 | 63,7 | 2,1 |
| 70,3 | 9,26 | 28,1 | 70,8 | 1,1 |

Ces données montrent qu'avec une perte de près de 50 % de la charge initiale, la tension de vapeur a changé de moins de 14 %. Avec une telle variation, le mélange ne peut pas être considéré comme pseudo-azéotrope. Toutefois, ces données montrent également que pour une fuite de moins de 40 %, même si la pression décroit (- 9 %), les concentrations en phase vapeur au cours de la fuite restent particulièrement stables (variations inférieures à 5,5 %) et varient fortement ensuite.

D'autre part, les valeurs maximales admissibles en HFC 32 afin que les mélanges restent ininflammables à température ambiante dans les mélanges 32/134a et 32/218 sont respectivement de 56 % en poids et de 67 % en poids.

Les mélanges ternaires selon l'invention sont donc ininflammables et le restent même lors d'une fuite de vapeur puisque la teneur maximale en HFC 32 est de 47,6 %.

### EXEMPLE 2

Cet exemple montre que la tension de vapeur des mélanges non azéotropiques de HFC 32/HFC 134a/FC 218 est proche de celle du HCFC 22 et ce sur une large gamme de température.

Le tableau 2 rassemble les données pour un mélange contenant en masse 33 % de HFC 32, 64 % de HFC 134a et 3 % de FC 218.

**TABLEAU 2**

| **Température (°C)** | **Pression absolue (bar)** | |
|---|---|---|
| | **Mélange 32/134a/218** | **HCFC 22** |
| -30 | 1,75 | 1,63 |
| -15 | 3,38 | 2,96 |
| 0 | 5,61 | 4,98 |
| 15 | 8,82 | 7,89 |
| 30 | 13,32 | 11,92 |
| 45 | 19,34 | 17,29 |
| 60 | 27,10 | 24,26 |

### EXEMPLE 3

Cet exemple illustre l'utilisation des mélanges selon l'invention comme fluides frigorigènes.

Les performances thermodynamiques de différents mélanges ternaires selon l'invention ont été comparées aux performances des constituants seuls, à celles de leurs mélanges binaires et à celles du HCFC 22 pour un cycle thermodynamique standard défini comme suit :

| | |
|---|---|
| Température de condensation | + 43°C |
| Température d'évaporation | + 7°C |
| Sous-refroidissement liquide | - 5°C |
| Surchauffe des vapeurs | + 11°C |

Le tableau 3 résume les performances thermodynamiques observées dans ces conditions pour le HFC 32, FC 218, HFC 134a et leurs mélanges.

**TABLEAU 3**

| **Composition (% masse) HFC 32/HFC 134a/FC 218** | **Glissement température °C** | **COP *** | **Capacité frigorifique volumétrique *** | **Différence température refoulement * °C** | **Différence pression condensation* bar** |
|---|---|---|---|---|---|
| 100/0/0 | 0 | 0,933 | 1,57 | + 17 | + 10,3 |
| 0/100/0 | 0 | 1,021 | 0,66 | - 14 | - 5,5 |
| 0/0/100 | 0 | 0,880 | 0,67 | - 27 | - 2,5 |
| 25/75/0 | 5,8 | 0,979 | 0,89 | - 5 | - 1,3 |
| 30/70/0 | 6,2 | 0,974 | 0,93 | - 3 | - 0,5 |
| 35/65/0 | 6,5 | 0,969 | 0,98 | - 2 | - 0,3 |
| 38/0/62 | 0 | 0,802 | 1,42 | - 8 | + 14,5 |
| 0/25/75 | 0 | 0,897 | 0,80 | - 23 | - 0,5 |
| 0/70/30 | 9,2 | 0,952 | 0,75 | - 17 | - 2,8 |
| 30/60/10 | 13,7 | 0,940 | 0,99 | - 4 | + 1,2 |
| 25/65/10 | 13,1 | 0,946 | 0,94 | - 5,5 | + 0,2 |
| 30/65/5 | 10,8 | 0,956 | 0,96 | - 3,5 | + 0,3 |
| 35/60/5 | 11,1 | 0,951 | 1,01 | - 2 | + 1,2 |
| 32/65/3 | 9,3 | 0,960 | 0,97 | - 2,7 | + 0,3 |
| 27/67,5/5,5 | 10,9 | 0,957 | 0,93 | - 4,5 | - 0,1 |
| 25/70/5 | 10,3 | 0,962 | 0,91 | - 5 | - 0,5 |

| | | | | | |
|---|---|---|---|---|---|
| * Relativement au HCFC 22 | | | | | |

Dans le même cycle thermodynamique défini précédemment, le mélange ternaire constitué de 31 % de HFC 32, 68 % de HFC 134a et 1 % de FC 14 présente les performances suivantes (∗relativement au HCFC 22) :
- Glissement de température : 16,8°C
- COP *: 0,943
- Capacité frigorifique volumétrique* : 0,96
- Différence * de température de refoulement : -1,5°C
- Différence * de pression de condensation : 0,45 bar

## Revendications

1. Mélange non azéotropique contenant en masse 26 à 34 % de difluorométhane, 56 à 73 % de 1,1,1 ,2-tétrafluoroéthane et 1 à 10 % de tétrafluorométhane ou d'octafluoropropane.

2. Mélange selon la revendication 1 contenant en masse, 26 à 34 % de difluorométhane, 56 à 73 % de 1,1,1,2-tétrafluoroéthane et 1 à 10 % d'octafluoropropane.

3. Mélange selon la revendication 2 contenant, en masse, environ 32 % de difluorométhane, 65 % de 1,1,1,2-tétrafluoroéthane et 3 % d'octafluoropropane.

4. Utilisation d'un mélange selon l'une des revendications 1 à 3 comme fluide frigorigène pour le conditionnement d'air.

## Claims

1. Nonazeotropic mixture containing by mass from 26 to 34% of difluoromethane, from 56 to 73% of 1,1,1,2-tetrafluoroethane and from 1 to 10% of tetrafluoromethane or octafluoropropane.

2. Mixture according to Claim 1, containing by mass from 26 to 34% of difluoromethane, from 56 to 73% of 1,1,1,2-tetrafluoroethane and from 1 to 10% of octafluoropropane.

3. Mixture according to Claim 2, containing by mass approximately 32% of difluoromethane, 65% of 1,1,1,2-tetrafluoroethane and 3% of octafluoropropane.

4. Use of a mixture according to one of Claims 1 to 3 as a refrigerant fluid for air conditioning.

## Patentansprüche

1. Nichtazeotrope Mischung, enthaltend in Masse 26 bis 34 % Difluormethan, 56 bis 73 % 1,1,1,2,-Tetrafluorethan und 1 bis 10% Tetrafluormethan oder Octafluorpropan.

2. Mischung nach Anspruch 1, enthaltend in Masse 26 bis 34 % Difluormethan, 56 bis 73 % 1,1,1,2-Tetrafluorethan und 1 bis 10 % Octafluorpropan.

3. Mischung nach Anspruch 2, enthaltend in Masse etwa 32 % Difluormethan, 65 % 1,1,1,2-Tetrafluorethan und 3 % Octafluorpropan.

4. Verwendung einer Mischung nach einem der Ansprüche 1 bis 3 als Kühlmittelflüssigkeit für die Luftklimatisierung.
